# EUROPEAN PATENT APPLICATION

(11) **EP 1 859 729 A1**
(43) Date of publication of application: **28.11.2007**
(21) Application number: 06715630.7
(22) Date of filing: 14.03.2006
(51) Int. Cl.: A61B 5/00

(54) **LIFESTYLE IMPROVEMENT SUPPORT SYSTEM AND LIFESTYLE IMPROVEMENT SUPPORT METHOD**

(30) Priority: 17.03.2005 JP 2005078162
(71) Applicant: MATSUSHITA ELECTRIC INDUSTRIAL CO., LTD., Osaka 571-8501 (JP)
(72) Inventor: TANIIKE, Yuko, c/o Matsushita Electric Ind. Co. Ltd., Shiromi, Chuo-ku, Osaka-shi, Osaka, 540-6207 (JP); KENJOU, Noriko, c/o Matsushita Electric Ind. Co. Ltd., Shiromi, Chuo-ku, Osaka-shi, Osaka, 540-6207 (JP); NOGUCHI, Eiji, c/o Matsushita Electric Ind. Co. Ltd., Shiromi, Chuo-ku, Osaka-shi, Osaka, 540-6207 (JP); KAWAMURA, Tatsurou, c/o Matsushita Electric Ind. Co. Ltd., Shiromi, Chuo-ku, Osaka-shi, Osaka, 540-6207 (JP)
(74) Representative: Pautex Schneider, Nicole Véronique
(86) International application number: PCT/JP2006/304958
(87) International publication number: WO 2006/098298

(57) **Abstract**

An object of the present invention is to provide a lifestyle improvement support system that can impart incentive information, such as points, with accuracy.

A lifestyle improvement support system according to the present invention includes: a measurement unit (12) which measures first biological information, which is biological information of a patient and which is reported by the patient; an information input unit (41) which inputs second biological information, which is biological information of the patient and is reported by a third-party institution, and which has a correlation with the first biological information; an incentive information creation unit which creates incentive information, which is information for increasing the incentive for the patient to improve his/her lifestyle, based on the first biological information and the second biological information; and a display unit which displays the incentive information.

## Description

### Technical Field

The present invention relates to a system that supports improvements in a person's lifestyle, and particularly relates to technology used when awarding incentive information, such as points, to a user of the system.

### Background Art

Alimentotherapy and ergotherapy are two of the fundamentals in the treatment of lifestyle-related disease. However, patients must constantly pay attention to implementing these treatments in their daily lives, and therefore it is difficult to continue such treatment.

In recent years, technology has been proposed in which amounts of exercise are graded, with points being added for performing predetermined target exercises within a set period of time and points being subtracted for failing to perform these exercises, with the goal of preventing users from failing to continue exercises (for example, see Patent Reference 1). In addition, a pedometer has been proposed in which characters corresponding to exercise amounts are displayed with the goal of causing a user to continue exercise for a long period of time while enjoying him/herself; the character develops in accordance with the user's progress, with the physique and expressions of the character changing depending on how long exercises are continued, the length of discontinuations, and so on (for example, see Patent Reference 2).

However, users are not provided with any concrete rewards when using the pedometer with this function, and thus users may not feel a sense of satisfaction. In the end, there are many cases in which users grow bored with repeating the same exercises and cannot attain a sense of achievement through continuing their daily exercises.

Accordingly, the health device proposed in Patent Reference 3 awards points to a user when there has been activity which maintains or improves the user's health or when there has been an improvement in measured values; for example, points are awarded for continuing to perform appropriate exercises, performing an effective amount of exercise, measuring biological information at a set date and time, taking measurements and acting in accordance with a doctor's advice, and so on. When these points exceed a predetermined amount, the user can receive a product, a service, or the like.
Patent Reference 1: Japanese Laid-Open Patent No. 11-178967
Patent Reference 2: Japanese Laid-Open Patent Application No. 11-52834
Patent Reference 3: Japanese Laid-Open Patent Application No. 2003-141260

### Disclosure of Invention

### Problems that Invention is to Solve

However, with the aforementioned conventional technology, it is difficult to ascertain whether the user him/herself has performed exercise, measurements, and so on using the health device. For example, points are awarded based on measurement results even when a member of the user's family, rather than the user him/herself, performs the measurement. Therefore, there is a problem in that the correct points cannot necessarily be awarded to the user.

An object of the present invention, which has been conceived in light of the aforementioned problem with the conventional technology, is to provide a lifestyle improvement support system that can award incentive information, such as points, with accuracy.

### Means to Solve the Problems

In order to achieve the object, a lifestyle improvement support system according to the present invention includes: a first biological information acquisition unit which acquires first biological information, which is biological information of a patient and which is reported by the patient; a second biological information acquisition unit which acquires second biological information, which is biological information of the patient and is reported by a third-party institution, and which has a correlation with the first biological information; an incentive information creation unit which creates incentive information, which is information for increasing the incentive for the patient to improve his/her lifestyle, based on the first biological information and the second biological information; and an output unit which outputs the incentive information.

In addition, a lifestyle improvement support method according to the present invention includes: a first biological information acquisition step of acquiring first biological information, which is biological information of a patient and which is reported by the patient; a second biological information acquisition step of acquiring second biological information, which is biological information of the patient and is reported by a third-party institution, and which has a correlation with the first biological information; an incentive information creation step of creating incentive information, which is information for increasing the incentive for the patient to improve his/her lifestyle, based on the first biological information and the second biological information; and an output step of outputting the incentive information.

In addition, a program for supporting improvements in lifestyle according to the present invention is for causing a computer to execute: a first biological information acquisition step of acquiring first biological information, which is biological information of a patient and which is reported by the patient; a second biological information acquisition step of acquiring second biological information, which is biological information of the patient and is reported by a third-party institution, and which has a correlation with the first biological information; an incentive information creation step of creating incentive information, which is information for increasing the incentive for the patient to improve his/her lifestyle, based on the first biological information and the second biological information; and an output step of outputting the incentive information.

### Effects of the Invention

The lifestyle improvement support system of the present invention makes it possible to award incentive information with accuracy.

### Brief Description of Drawings

FIG. 1 is a block diagram showing a simplified configuration of the lifestyle improvement support system according to the first embodiment of the present invention.
FIG. 2 is a block diagram showing a detailed configuration of a server apparatus according to the first embodiment of the present invention.
FIG. 3 is a flowchart showing a process of the lifestyle improvement support system according to the first embodiment of the present invention.
FIG. 4 is a diagram showing an example of data stored in a biological information storage unit in the first embodiment of the present invention.
FIG. 5 is a diagram showing an example of data stored in a points/bills storage unit in the first embodiment of the present invention.
FIG. 6 is a diagram showing an example of a notification screen that shows tentative points and advice in the first embodiment of the present invention.
FIG. 7 is a diagram showing an example of a notification screen that shows finalized points and incentives in the first embodiment of the present invention.
FIG. 8 is a diagram showing another example of data stored in a points/bills storage unit in the first embodiment of the present invention.
FIG. 9 is a diagram showing an example of a notification screen that shows incentives in the first embodiment of the present invention.
FIG. 10 is a flowchart showing a process of finalizing points in the first embodiment of the present invention.
FIG. 11 is a block diagram showing a simplified configuration of the lifestyle improvement support system according to the second embodiment of the present invention.
FIG. 12 is a diagram showing an example of data stored in a points/bills storage unit in the second embodiment of the present invention.
FIG. 13 is a flowchart showing a process of the lifestyle improvement support system according to the second embodiment of the present invention.
FIG. 14 is a flowchart showing a process of another lifestyle improvement support system according to the second embodiment of the present invention.
FIG. 15 is a flowchart showing a process of another lifestyle improvement support system according to the second embodiment of the present invention.
FIG. 16 is a diagram showing an example of a notification screen that shows finalized points and incentives in the second embodiment of the present invention.
FIG. 17 is a diagram showing another example of data stored in a points/bills storage unit in the second embodiment of the present invention.
FIG. 18 is a diagram showing an example of a notification screen that shows finalized points and advice in the second embodiment of the present invention.
FIG. 19 is a flowchart showing a process in which a lifestyle improvement support system according to the third embodiment of the present invention awards points to a clinic.
FIG. 20 is a diagram showing an example of data stored in a points/bills storage unit in the third embodiment of the present invention.

### Numerical References

- 1: Client Terminal
- 2: Communications Network
- 3: Server Apparatus
- 4: Clinic Terminal
- 10, 40: Power Button
- 11, 41: Information Input Unit
- 12: Measurement Unit
- 13, 43: Display Unit
- 14, 44: CPU
- 15, 31, 45: Sending/Receiving Unit
- 16, 46: Information Storage Unit
- 31: Sending/Receiving Unit
- 32: CPU
- 33: Information Storage Unit
- 310: Sending/Receiving Unit
- 320: Incentive Information Creation Unit
- 321: Tentative Unit
- 322: Revision Unit
- 330: Advice Information Creation Unit
- 340: Correlative Relationship Storage Unit
- 350: Biological Information Storage Unit
- 360: Points/Bills Storage Unit

### Best Mode for Carrying Out the Invention

A lifestyle improvement support system according to the present invention includes: a first biological information acquisition unit which acquires first biological information, which is biological information of a patient and which is reported by the patient; a second biological information acquisition unit which acquires second biological information, which is biological information of the patient and is reported by a third-party institution, and which has a correlation with the first biological information; an incentive information creation unit which creates incentive information, which is information for increasing the incentive for the patient to improve his/her lifestyle, based on the first biological information and the second biological information; and an output unit which outputs the incentive information. Through this, incentive information is created based not only on the first biological information reported by the patient, but also on the second biological information reported by a third-party institution, which makes it possible to accurately provide the incentive information to the patient.

In addition, the incentive information creation unit creates tentative incentive information, which is the incentive information that has not yet been finalized, based on the first biological information, and after that, creates finalized incentive information, which is the incentive information that has been finalized, based on the tentative incentive information and the second biological information. Through this, when the tentative incentive information is outputted by an output unit, evaluation is performed without negating the patient's effort to improve his/her lifestyle, which has the effect of causing the patient to continue his/her lifestyle improvement. On the other hand, when the finalized incentive information is outputted by the output unit, accurately-awarded incentive information is presented to the patient; therefore, it is possible to provide higher incentives to the patient in the case where the first biological information is that of the patient him/herself.

In addition, the lifestyle improvement support system further includes a correlative relationship storage unit which stores the correlative relationship between the first biological information and the second biological information, and the incentive information creation unit finds the estimated value of the second biological information based on the correlative relationship and the first biological information, and creates the finalized incentive information so that the incentive for the patient to improve his/her lifestyle increases the lower the degree of divergence is between the second biological information and the estimated value of the second biological information. Through this, an estimated value can be obtained in an accurate manner for sicknesses which have clear correlative relationships, and thus the lower the degree of falsity in the first biological information, the lower the degree of divergence between the estimated value and the second biological information. In other words, the lower the degree of divergence is, the higher the incentive is for the patient, and thus it can be expected that the patient will work more actively to improve his/her lifestyle.

In addition, the lifestyle improvement support system further includes a target value storage unit which stores the target value of the second biological information, and the incentive information creation unit creates the finalized incentive information so that the incentive for the patient to improve his/her lifestyle increases the higher the degree of achievement is between the second biological information and the target value of the second biological information. Through this, it is possible to provide the patient with higher incentives when daily lifestyle improvement has a positive effect on treatment. In other words, the higher the degree of achievement is between the second biological information and the target value of the second biological information, the higher the incentive is for the patient, and thus it can be expected that the patient will work more actively to improve his/her lifestyle.

In addition, the lifestyle improvement support system further includes a biological information storage unit which stores the second biological information, and the incentive information creation unit creates the finalized incentive information so that the incentive for the patient to improve his/her lifestyle increases the higher the degree of improvement is between the present second biological information and the previous second biological information. Through this, it is possible to provide the patient with higher incentives when daily lifestyle improvement has a positive effect on treatment. In other words, the higher the degree of improvement is between the present second biological information and the previous second biological information, the higher the incentive is for the patient, and thus it can be expected that the patient will work more actively to improve his/her lifestyle.

In addition, the lifestyle improvement support system further includes an advice information creation unit which creates advice information, which is information indicating advice for the patient regarding improvement in his/her lifestyle, based on the first biological information, and the output unit outputs the advice information. Through this, the patient receives specific advice tailored to him/herself, making it possible to achieve greater results in lifestyle improvement.

In addition, the incentive information creation unit generates third-party institution incentive information, which is information for increasing the incentive for efforts on the part of the third-party institution, based on the incentive information, and the output unit outputs the third-party institution incentive information. Through this, it is possible to display, to the patient, incentive information in response to effort on the part of a third-party institution.

In addition, the lifestyle improvement support system further includes a client terminal which belongs to the patient and a third-party institution terminal which belongs to the third-party institution. The client terminal includes: a first biological information acceptance unit which accepts input of the first biological information; and a first biological information sending unit which sends the first biological information accepted by the first biological information acceptance unit to the first biological information acquisition unit via a network. The third-party terminal includes: a second biological information acceptance unit which accepts input of the second biological information; and a second biological information sending unit which sends the second biological information accepted by the second biological information acceptance unit to the second biological information acquisition unit via a network. Through this, information is sent and received in real time between the client terminal, the third-party institution, and the server apparatus, and therefore the patient can obtain information instantly.

In addition, the lifestyle improvement support system further includes a client terminal which belongs to the patient and a third-party institution terminal which belongs to the third-party institution. The client terminal includes: a first biological information acceptance unit which accepts input of the first biological information; and a first biological information sending unit which sends the first biological information accepted by the first biological information acceptance unit to the first biological information acquisition unit via a storage medium. The third-party terminal includes: a second biological information acceptance unit which accepts input of the second biological information; and a second biological information sending unit which sends the second biological information accepted by the second biological information acceptance unit to the second biological information acquisition unit via a network. Through this, it is possible for the patient to utilize the present system even in the case where the client terminal is not connected to a network such as the Internet.

In addition, the first biological information is information that indicates results of the patient's lifestyle improvement. Through this, it becomes possible to use, in the present system, information such as an amount of exercise, which is not a substance present within the patient's body per se, but is information that indicates results of the patient's lifestyle.

Note that the present invention may be realized not only as this type of lifestyle improvement support system, but also as a lifestyle improvement support method which implements the characteristic units of the lifestyle improvement support system as steps, and also as a program that causes a computer to execute those steps. It goes without saying that such a program may be distributed via a storage medium such as a CD-ROM, a transmission medium such as the Internet, and so on.

Hereafter, embodiments of the present invention shall be described in detail using the diagrams.

### (First Embodiment)

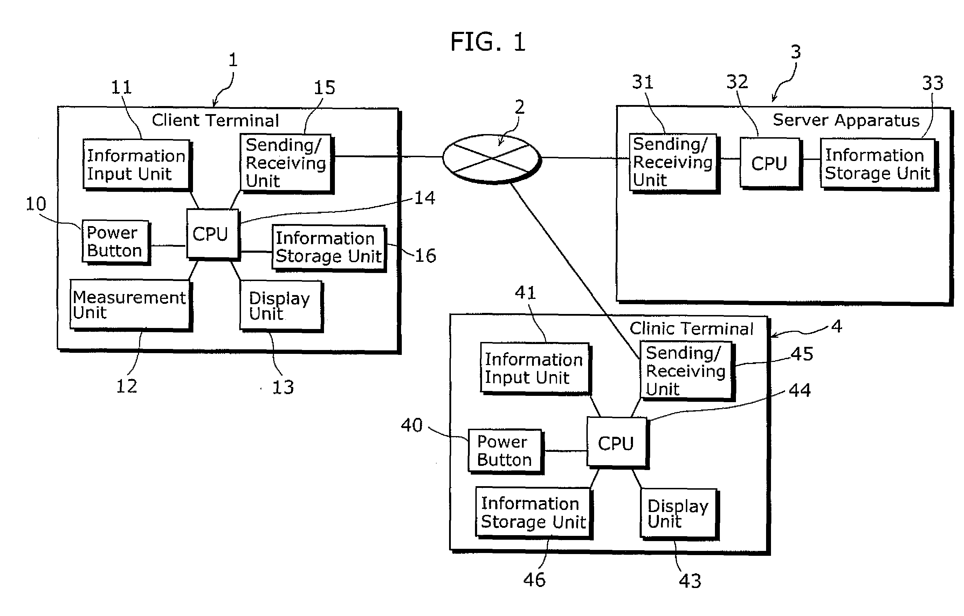

FIG. 1 is a block diagram showing a simplified configuration of the lifestyle improvement support system according to the first embodiment of the present invention. This system supports improvement in a person's lifestyle; here, the system includes a client terminal 1, a server apparatus 3, and a clinic terminal 4, which is a third-party institution terminal, all of which are connected by a communications network 2, such as an ADSL network, a cellular network, or the like.

The client terminal 1 is in a patient's possession, and includes a power button 10, an information input unit 11, a measurement unit 12, a display unit 13, a CPU 14, a sending/receiving unit 15, and an information storage unit 16. The power button 10 is a button used to turn the power of the terminal 1 on and off. The information input unit 11 is a keyboard or the like for inputting information. The measurement unit 12 is a disposable measurement chip or the like that can quantitatively measure the salt content of urine, and corresponds to the first biological information acceptance unit of the present invention. The display unit 13 is a display or the like for displaying incentive information, advice information, or the like, which shall be described later. The CPU 14 is a central processing unit which performs various processes related to the lifestyle improvement support system. The sending/receiving unit 15 is a communications board or the like that sends/receives information to/from the server apparatus 3, and corresponds to the first biological information sending unit of the present invention. The information storage unit 16 is a hard disk or the like that stores information such as a patient ID and so on.

The server apparatus 3 is an apparatus that possesses the means to provide the service implemented by the present system (hereafter referred to simply as "service"), and includes a sending/receiving unit 31, a CPU 32, and an information storage unit 33. The sending/receiving unit 31 is a communications board or the like that sends/receives information to/from the client terminal 1 and so on, and corresponds to the first biological information acquisition unit, second biological information acquisition unit, and output unit of the present invention. The CPU 32 is a central processing unit which performs various processes related to the lifestyle improvement support system. The information storage unit 33 is a hard disk or the like that stores various types of information, such as biological information.

The clinic terminal 4 is a terminal possessed by a third-party institution such as a clinic, and includes a power button 40, an information input unit 41, a display unit 43, a CPU 44, a sending/receiving unit 45, and an information storage unit 46. The power button 40 is a button used to turn the power of the terminal 4 on and off. The information input unit 41 is a keyboard or the like for inputting information, and corresponds to the second biological information acceptance unit of the present invention. The display unit 43 is a display or the like for displaying examination results and the like, which shall be described later. The CPU 44 is a central processing unit which performs various processes related to the lifestyle improvement support system. The sending/receiving unit 45 is a communications board or the like for sending/receiving information to/from the server apparatus 3, and corresponds to the second biological information sending unit of the present invention. The information storage unit 46 is a hard disk or the like that stores information such as a clinic ID and so on.

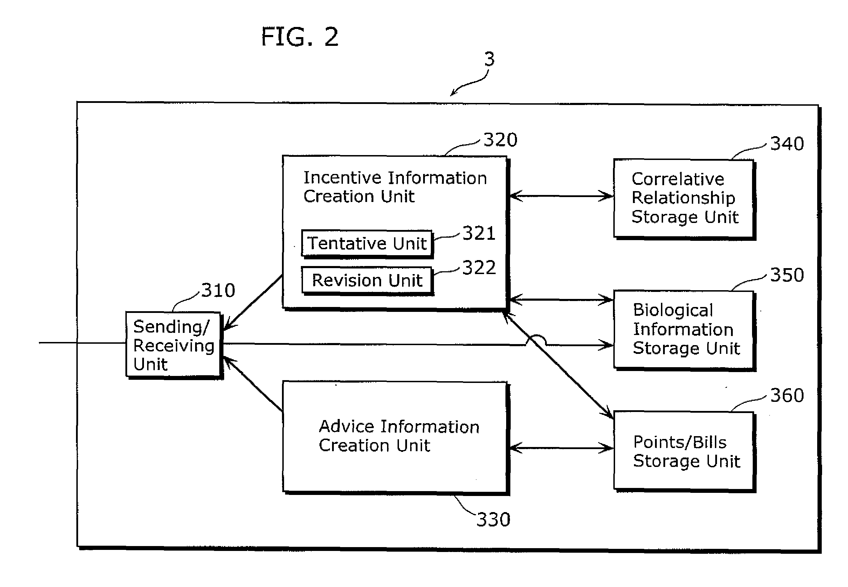

FIG. 2 is a diagram showing the configuration of the server apparatus 3 in detail. As shown in FIG. 2, the server apparatus 3 functionally includes a sending/receiving unit 310, an incentive information creation unit 320, an advice information creation unit 330, a correlative relationship storage unit 340, a biological information storage unit 350, and a points/bills storage unit 360.

The sending/receiving unit 310 is a means that functionally expresses the sending/receiving unit 31 (refer to FIG. 1). The incentive information creation unit 320 corresponds to the incentive information creation unit of the present invention, and creates incentive information. "Incentive information" is information for increasing incentives to a patient who improves his/her lifestyle; points awarded to the patient is one example. The incentive information creation unit 320 includes a tentative unit 321, which creates tentative incentive information, and a revision unit 322, which revises the tentative incentive information. When the revision unit 322 revises the tentative incentive information, finalized incentive information is created. The advice information creation unit 330 corresponds to the advice information creation unit of the present invention, and creates advice information. "Advice information" refers to information that indicates advice given to the patient regarding his/her lifestyle improvement. The correlative relationship storage unit 340 corresponds to the correlative relationship storage unit of the present invention, and stores a correlative relationship between measurement results and points (explained later), a correlative relationship between first biological information and second biological information, and so on. Here, "first biological information" refers to the patient's biological information, such as the salt content of his/her urine, as reported by the patient him/herself; "second biological information" refers to the patient's biological information, such as blood pressure readings, as reported by a third-party institution. There is a correlative relationship between the first biological information and the second biological information (to be described later). The biological information storage unit 350 corresponds to the biological information storage unit of the present invention, and stores biological information, such as the first biological information, the second biological information, and so on. The points/bills storage unit 360 stores information regarding points, bills, and so on.

Lifestyle-related disease patients work towards improving their lifestyles with a doctor's guidance and with certain goals. In particular, patients with hypertension follow dietary guidance that places restrictions on their salt intake. It is possible to valuate the amount of salt ingested at mealtime from the amount of salt in urine expelled by the patient. In the present embodiment, the patient measures the salt content of his/her urine every day at a specified time, and the results of that measurement are sent from the client terminal 1 to the server apparatus 3. The server apparatus 3 judges the status of improvement in the patient's lifestyle, converts the judgment results to points, and returns the resultant back to the client terminal 1. Through this, it is possible for the patient to reflect on what he/she has eaten on a daily basis.

An example of awarding points shall be given here. Assuming that the target salt concentration is, for example, 2g/l, when measuring a first salt concentration of urine in the early morning, five points are awarded if the measured salt concentration is within 2g/l, three points are awarded if the salt concentration is within 4g/l, and one point is awarded if the salt concentration is within 6g/l. Furthermore, assuming that the target number for measurement frequency is three times per week, five points are awarded if the measurement frequency is four or more times in a week, three points are awarded if the measurement frequency is three times a week, and one point is awarded if the measurement frequency is one to two times a week. Further still, five points are awarded as bonus points if the measurement frequency exceeds twelve times in a month. The correlative relationship between the measurement results/measurement frequency of the first salt concentration of urine in the early morning and points is stored in the correlative relationship storage unit 340. The incentive information creation unit 320 of the server apparatus 3 coverts the measurement results and so on into points based on this correlative relationship.

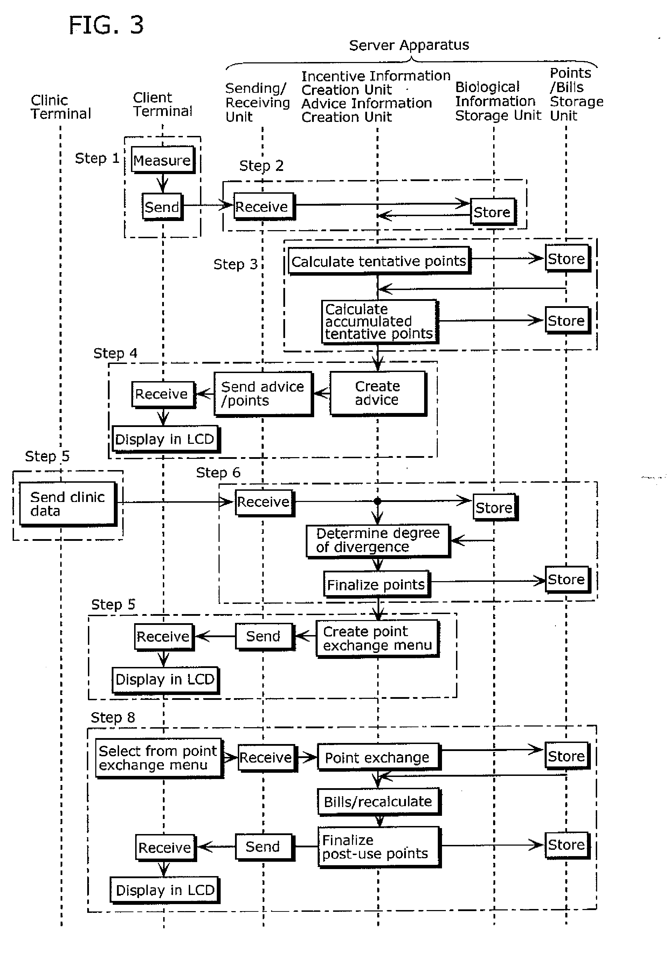

FIG. 3 is a flowchart showing a process of the lifestyle improvement support system according to the first embodiment of the present invention. Hereafter, the process of this lifestyle improvement support system shall be described using FIGs. 1 to 3.

The patient takes a daily measurement using the measurement unit 12, and the obtained measurement results are sent from the client terminal 1 to the server apparatus 3 (Step 1). In addition to the measurement results, the sending terminal information, the measurement date, and the measurement frequency are included in the information sent. The terminal information is, for example, a patient ID, which is information for identifying the patient; this patient ID is stored, in advance, in the information storage unit 16 of the client terminal 1. In this manner, information is sent and received using the patient ID, and thus the present lifestyle improvement support system utilizes a scheme which gives extra attention to personal information leaks. The measurement date is information indicating the date on which the salt concentration of the patient's urine was measured. The measurement frequency is information indicating the frequency at which the salt concentration of the patient's urine was measured, and can indicate the number of measurements within a specified period, such as, for example, once in the first week of September.

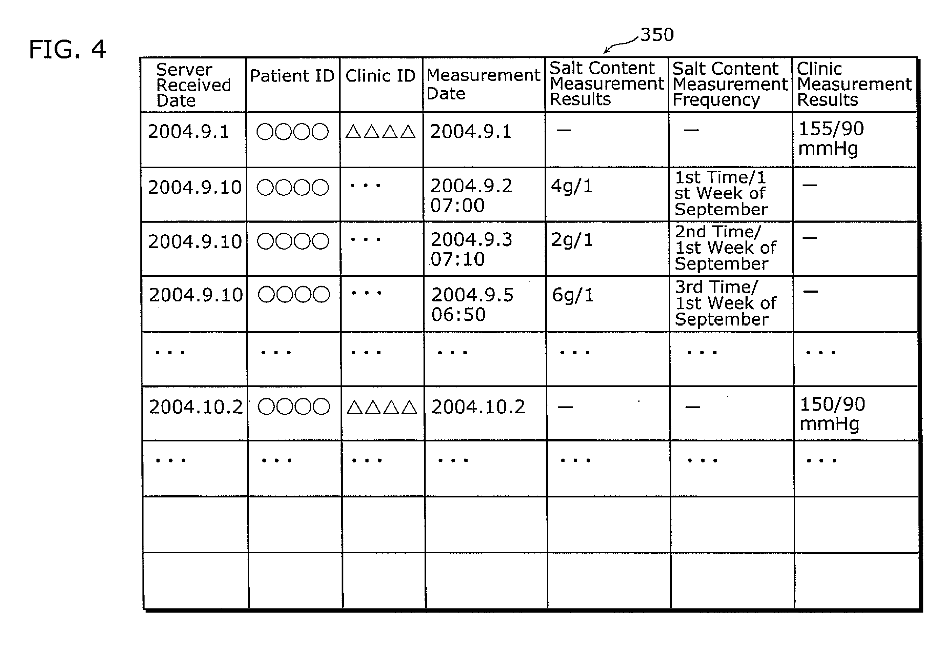

The information sent by the client terminal 1 is received by the sending/receiving unit 31 of the server apparatus 3 and stored in the biological information storage unit 350 (Step 2). FIG. 4 is a diagram showing an example of data stored in the biological information storage unit 350. As shown in FIG. 4, the biological information storage unit 350 stores a server received date, patient ID, clinic ID, measurement date, salt measurement result, salt measurement frequency, and clinic measurement result in association with one another.

Next, the tentative unit 321 within the server apparatus 3 converts the measurement results to points, and stores those points, as well as the points accumulated thus far at that point in time, in the points/bills storage unit 360 (Step 3). The points converted to here are tentative points, and thus have not been finalized. In addition, the tentative unit 321 within the server apparatus 3 converts the measurement frequency, sent along with the measurement results, into points, which are added to the tentative points obtained from the measurement results, resulting in the accumulated tentative points. At this time, the tentative points are determined based on the target salt concentration and the target measurement frequency. The target salt concentration and the target measurement frequency are sent from the clinic terminal 4 to the server apparatus 3, and are stored in the correlative relationship storage unit 340 of the server apparatus 3.

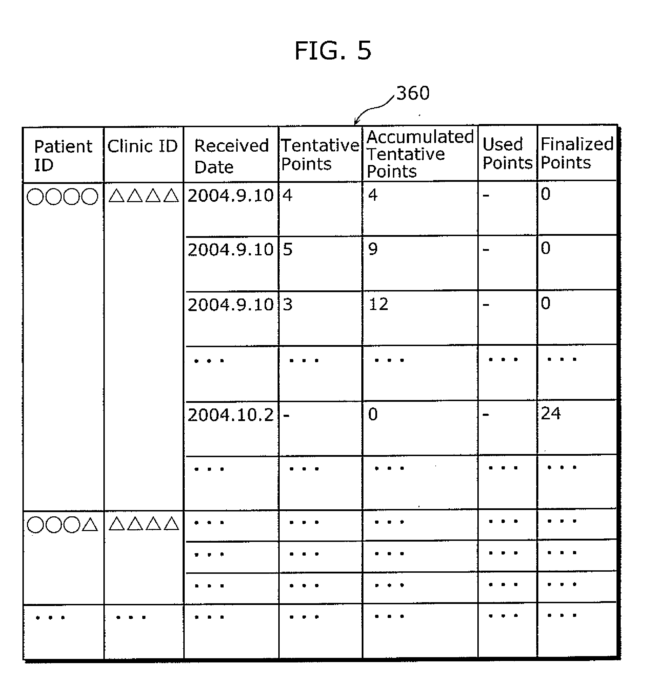

FIG. 5 is a diagram showing an example of data stored in the points/bills storage unit 360. As shown in FIG. 5, the points/bills storage unit 360 stores the patient ID, clinic ID, date received, tentative points, accumulated tentative points, used points, and finalized points in association with one another.

As shown in FIG. 4, the salt concentration measured on September 2, 2004 is 4g/l, and thus three points are awarded. As this measurement is the first measurement of the first week of September, an extra one point is awarded. Therefore, as can be seen in FIG. 5, a total of four points are awarded as tentative points for the measurement taken on September 2, 2004.

In the same manner, the salt concentration measured on September 3, 2004 is 2g/l, and thus five points are awarded. As this measurement is the second measurement of the first week of September, no points for measurement frequency are awarded. Therefore, a total of five points are awarded as tentative points for the measurement taken on September 3, 2004, and thus the number of accumulated tentative points is nine.

Next, the salt concentration measured on September 5, 2004 is 6g/l, and thus one point is awarded. As this measurement is the third measurement of the first week of September, an extra three points are awarded. Therefore, a total of four points are awarded as tentative points for the measurement taken on September 5, 2004, and thus the number of accumulated tentative points is thirteen. Through this, the number of accumulated tentative points for the measurements taken in the first week of September is thirteen.

The advice information creation unit 330 creates advice information (Step 4). Included in this advice is evaluation of the previous day's meals and goals for the current day's meals. For example, if the amount of salt ingested in the previous day's meals, determined by the results of measuring the salt content of the patient's urine, falls within a target value, the patient is praised for the content of the previous day's meal, which is suited to him/her, and is suggested to continue that kind of lifestyle. On the other hand, if the amount is significantly skewed from the target value, the patient is first praised for taking the measurement, and is then prompted to act in order to identify the cause (in other words, the patient is prompted to recall if there was something in the previous day's meal that increased the amount of salt ingested).

In this manner, accumulated tentative points are calculated and advice information is created each time measurement results are sent from the client terminal 1. The accumulated tentative points and advice information are sent by the sending/receiving unit 310 of the server apparatus 3 to the client terminal 1, and received by the sending/receiving unit 15 of the client terminal 1. Accordingly, a notification screen showing the tentative points and advice is displayed in the display unit 13 of the client terminal 1.

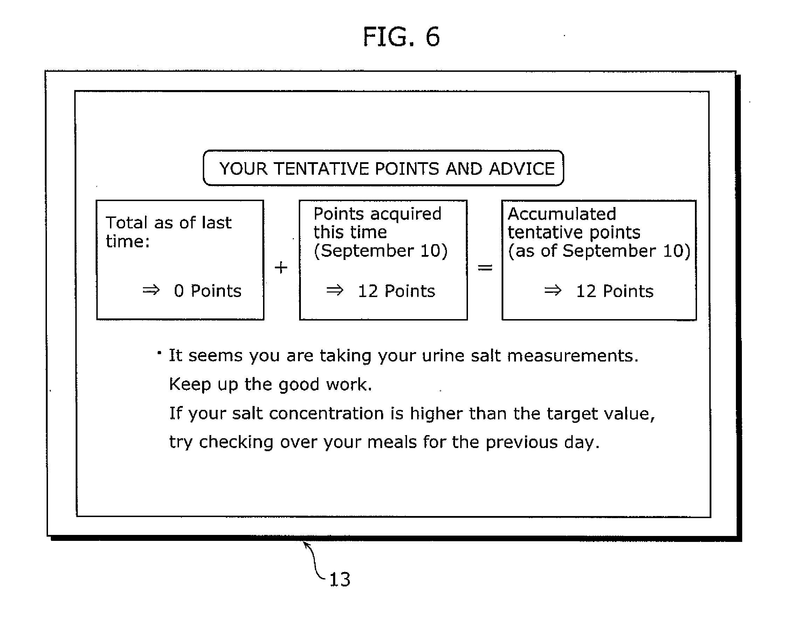

FIG. 6 is a diagram showing an example of the notification screen that shows the tentative points and advice. The accumulated tentative points acquired as of the last time, the tentative points acquired this time, the total of these, which is the current amount of accumulated tentative points, and the advice information are included in this notification screen. In other words, incentive information created based on the first biological information reported by the patient him/herself is also sent to the client terminal 1. Doing so evaluates the patient's lifestyle improvement without negating his/her effort, which has the effect of prompting the patient to continue improving his/her lifestyle. In addition, the incentive information, created based on the measurement frequency as well as the measurement results, is sent to the client terminal 1, and thus it can be expected that the patient's enthusiasm towards taking the measurements will increase.

The patient undergoes an examination at the clinic after continuing this lifestyle improvement for one month. A doctor sends the examination data (clinic data) from the clinic terminal 4, which is installed in the clinic, to the server 3 (Step 5). The "clinic data" referred to here is made up of the examination results, the patient ID, the measurement date, and the clinic ID; the "examination results" refers to a blood pressure reading taken during the examination at the clinic.

The clinic data sent from the clinic terminal 4 is received by the sending/receiving unit 31 of the server apparatus 3 and stored in the biological information storage unit 350. Here, the revision unit 322 of the server acquires, from the biological information storage unit 350, the blood pressure reading taken in the present examination (the present blood pressure reading) and the blood pressure reading taken in the previous examination (the previous blood pressure reading), and determines the degree to which the present blood pressure reading has improved over the previous blood pressure reading. For example, in the case where the patient suffers from hypertension, the lower the present blood pressure reading is in comparison to the previous blood pressure reading, the higher the degree of improvement. Based on the results of this determination, the tentative points accumulated by the patient throughout the past month are revised into finalized points, and the finalized points are stored in the points/bills storage unit 360 (Step 6). This makes it possible to award higher amounts of finalized points for higher degrees of improvement, and as a result, it can be expected that the patient will become more enthusiastic about improving his/her lifestyle.

Here, the tentative points have been revised into finalized points based on the degree of improvement; however, it should be noted that the present invention is not limited to such a setup. That is, in order to determine the accuracy of the points reported by the patient, it is preferable to revise the tentative points into finalized points based on the degree of divergence between the present blood pressure reading and an estimated blood pressure reading.

To be more specific, there are, among essential hypertension patients, hypertension patients with salt sensitivity, with whom a direct correlative relationship between salt ingestion and hypertension can be seen (Matsuoka, Komyo: Hypertension Navigator, 2000: Medical Review Inc., pp. 16-17). If the salt content of the patient's urine can be found from the correlation between (slope of) a decrease in the ingested amount of salt and a decrease in blood pressure, it is possible to estimate the results of the patient's examination at the clinic (the present blood pressure reading). Accordingly, the accuracy of the points awarded based solely on the urine salt content measurement results as reported by the patient is determined based on the degree of divergence between the present blood pressure reading reported by the clinic and the estimated blood pressure reading found from the urine salt content measurement results as reported by the patient. The operation of determining the accuracy corresponds to the operation for converting tentative points to finalized points of Step 6. As an example, when the present blood pressure reading is within the range {estimated blood pressure reading x 97% - estimated blood pressure reading x 103%}, the degree of divergence between the present blood pressure reading and the estimated blood pressure reading is determined to be low; on the other hand, when the present blood pressure reading is not within the range {estimated blood pressure reading x 97% - estimated blood pressure reading x 103%}, the degree of divergence is determined to be high. In the case where the degree of divergence is determined to be high, the acquired tentative points are applied directly as finalized points, and in the case where the degree of divergence is determined to be low, two times the amount of acquired tentative points are applied as finalized points.

Next, the finalized points and a menu of incentives for which the finalized points can be exchanged are sent to the client terminal 1 by the sending/receiving unit 310 of the server apparatus 3, and are received by the sending/receiving unit 15 of the client terminal 1. Accordingly, a notification screen showing the finalized points and incentives is displayed in the display unit 13 of the client terminal 1 (Step 7).

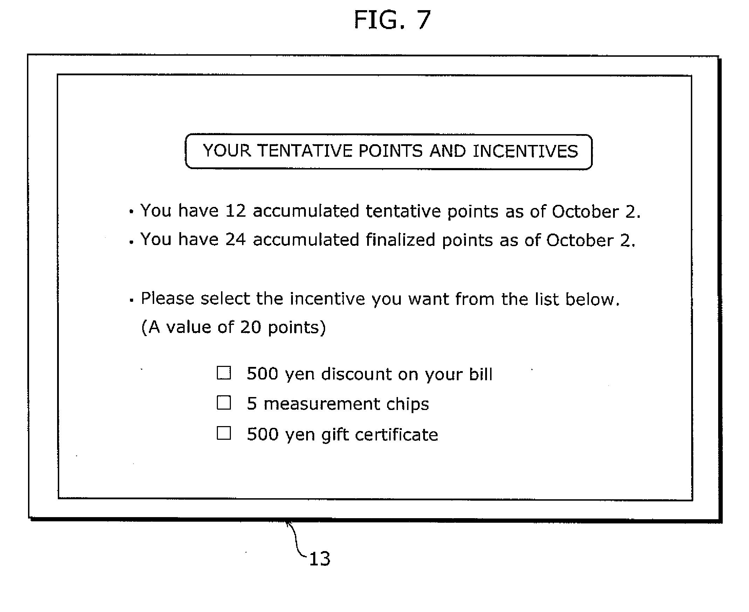

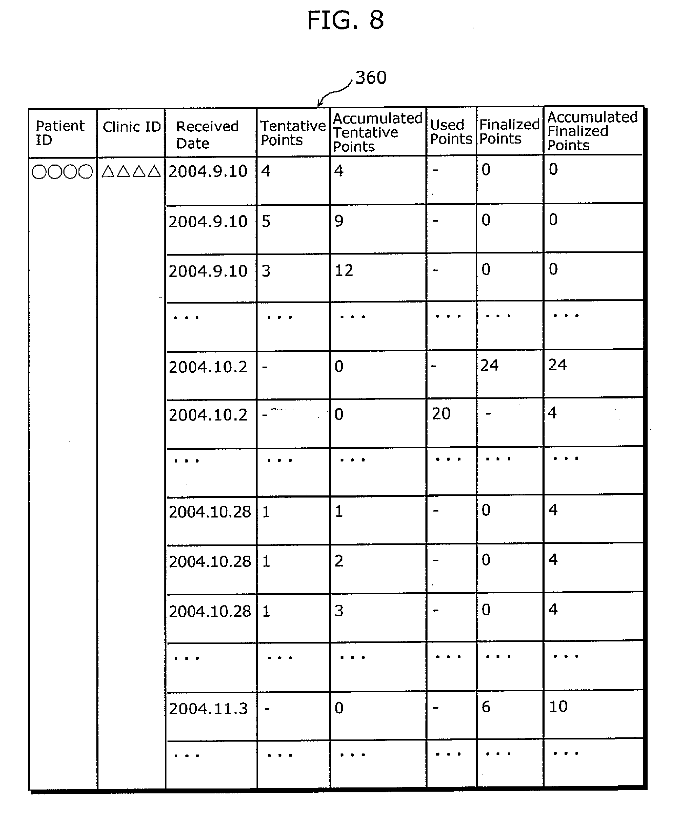

FIG. 7 is a diagram showing an example of a notification screen that shows finalized points and incentives. Finalized points and incentives are included in this notification screen. Of course, the accumulated tentative points acquired thus far may also be included. The finalized points may be newly-acquired finalized points, or they may be accumulated finalized points acquired thus far. The accumulated finalized points can be obtained by totaling the finalized points stored in the points/bifls storage unit 360. Alternatively, a configuration in which the accumulated finalized points are also stored in the points/bills storage unit 360 in addition to the finalized points may be employed, as shown in FIG. 8. Discounts on bills, free disposable measurement chips, gift certificates, and other prizes can be given as examples of incentives. The details of these incentives are displayed in the display unit 13, and therefore the patient uses the information input unit 11 to select the incentive he/she desires from among those displayed. Results of this selection are sent from the client terminal 1 to the server apparatus 3, and therefore the patient can thus receive an incentive that corresponds to the finalized points he/she acquired. At that time, a notification screen showing the incentive is displayed in the display unit 13 of the client terminal 1 (Step 8).

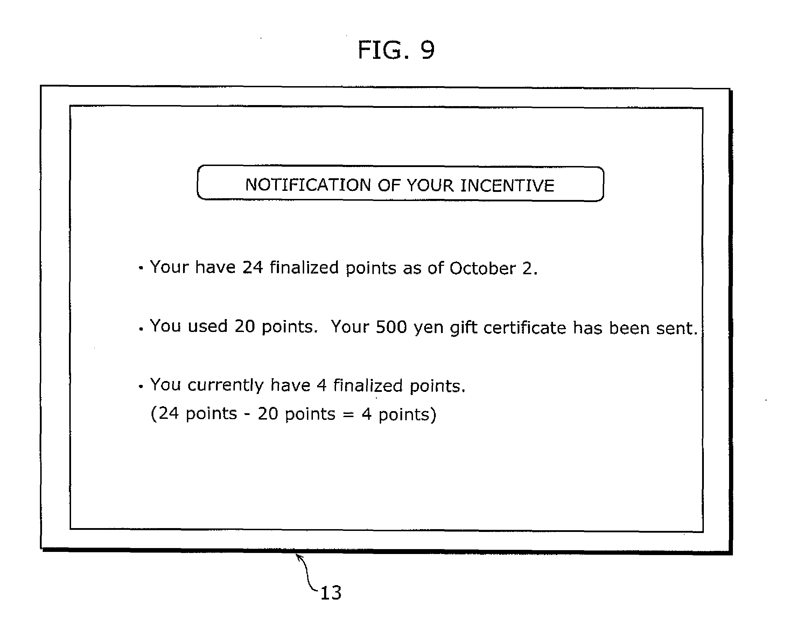

FIG. 9 is a diagram showing an example of a notification screen that shows the incentive. The incentive received by the patient and the finalized points present after receiving the incentive are included in this notification screen. Of course, the finalized points present before receiving the incentive may also be included. Through this, the patient can know when the incentive application process has finished, and can confirm that there are no mistakes in the details of this application.

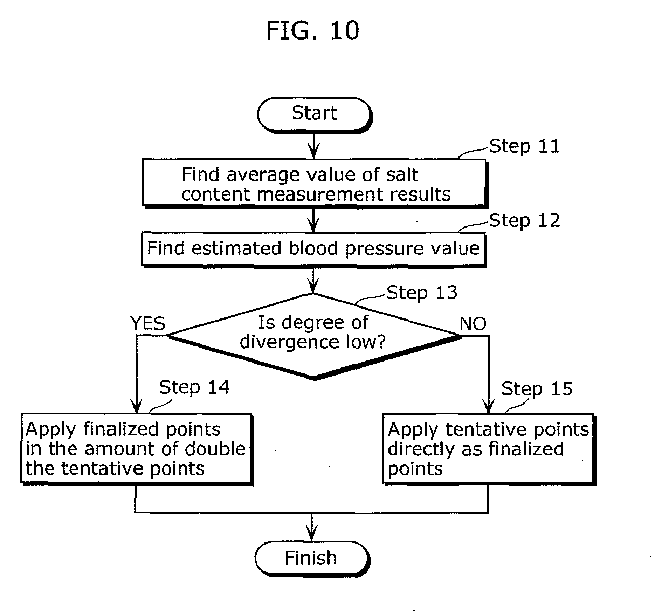

FIG. 10 is a flowchart showing a point finalization process. Hereafter, the method for finalizing the points mentioned earlier shall be described in detail using FIGs. 10, 4, and 5.

As shown in FIG. 4, the salt concentration measured on September 2, 2004 is 4g/l, the salt concentration measured on September 3, 2004 is 2g/l, and the salt concentration measured on September 5, 2004 is 6g/l; therefore, the average concentration of salt measured over these three days is 4g/l (Step 11). A decrease in the amount of salt ingested as compared to the previous month is identified using this average of 4g/l, and an estimated blood pressure reading is found based on the correlation (slope) between the decrease in the amount of salt ingested by the patient and the degree of decrease in blood pressure as stored in the correlative relationship storage unit 340. When the present blood pressure reading is within the range {estimated blood pressure reading x 97% - estimated blood pressure reading x 103%}, the degree of divergence is determined to be low, and finalized points in the amount of double the acquired tentative points are applied (Steps 13-14). For example, as shown in FIG. 5, when the final amount of accumulated tentative points for October 2, 2004 is twelve points, and the degree of divergence is determined to be low, the tentative points are doubled, and thus 24 points are applied as finalized points. On the other hand, when the present blood pressure reading is not within the range {estimated blood pressure reading x 97% - estimated blood pressure reading x 103%}, the degree of divergence is determined to be high, and the acquired tentative points are directly applied as finalized points (Steps 13-15). For example, when the final amount of accumulated tentative points for October 2, 2004 is twelve points, and the degree of divergence is determined to be high, these tentative points are directly applied as finalized points.

As described thus far, the lifestyle improvement support system of the present embodiment uses not only biological information sent from the client terminal 1 but also objective biological information sent from the clinic terminal 4 to create incentive information, thus making it possible to accurately award the incentive information. For example, when the patient's family member has taken measurements in place of the patient him/herself, the degree of divergence between the estimated blood pressure reading found from the urine salt content measurement results and the patient's blood pressure reading as measured in the clinic increases; this makes it possible to award a lower amount of finalized points. On the other hand, when the patient him/herself has taken the measurement, the degree of divergence decreases, which makes it possible to award a higher amount of finalized points.

With the conventional lifestyle improvement support system, points are determined based on the frequency and results of attempts to improve one's lifestyle; there is no means for determining points using the results of a doctor's examination. Therefore, there are situations where attempts at lifestyle improvement end in failure, or points become the main goal of lifestyle improvement; as a result, improving one's condition, which is the original goal of the lifestyle improvement support system, falls by the wayside.

In response to this, the present lifestyle improvement support system finalizes the points with reference to the results of a doctor's examination. Therefore, as mentioned earlier, it is possible to determine, using the results of the examination performed in a clinic, whether or not the person involved in the lifestyle improvement activities is truly the patient him/herself. In addition, because the improvement program is managed by a doctor, it is possible to set goals within a range effective in treatment.

Of course, as the patient feels that the effects of the daily lifestyle improvement activities are being reflected in his/her treatment, there is an effect in that the patient will pick up habits for improving his/her lifestyle earlier on. Moreover, as points and advice are presented to the patient in addition to the daily measurement results, there is an additional effect in that motivation to take the measurements can be continuously imparted on the patient. At this time, even supposing the measurement results from the previous day are unfavorable, points are nevertheless added, which prompts the patient to continue the measurements.

Furthermore, the client terminal 1 includes a measurement unit 12, such as a measurement chip or the like, which makes it easy to send the measurement results to the server apparatus 3. Accordingly, it can be expected that the patient will continue his/her daily measurements.

Although descriptions have been given here regarding the measurement of salt content in the patient's urine, it should be noted that the present invention is not limited to such a setup. In other words, any item may be used as the measurement target as long as it is an indicator of the results of the patient's lifestyle improvement and can be measured by the patient in his/her own home. For example, urine protein, urine sugar, blood sugar, blood pressure, weight, the number of steps walked, and so on may be measured. Of course, if the target to be measured changes on the patient's side, it is preferable to change the target to be measured on the clinic's side accordingly. For example, in the case where the patient suffers from diabetes and measures his/her blood sugar instead of urine salt content, it is preferable for the clinic to measure the patient's hemoglobin A1c rather than his/her blood pressure. The reason for this is that just as there is a correlative relationship between urine salt content and blood pressure, there is a correlative relationship between blood sugar and hemoglobin A1c. Therefore, it is clear that the same items should be measured on the patient's side and the clinic's side, rather than having different items measured.

In addition, although the results of measuring the concentration of urine salt are used here, the present invention is not limited to such a setup. For example, measurement results, in which the salt amount has been converted by performing urine amount correction, creatinine correction, or the like for the urine salt concentration, may be used as well. "Creatinine correction" refers to a method which measures the concentration of creatinine in the urine and uses the results of that measurement to calculate, based on the urine salt concentration, the amount of urine salt expelled in one day. In such a case, it is possible to utilize, as the amount of salt, the amount of urine salt expelled in a day, for example.

In addition, although the urine salt contained within the first urine expelled in the early morning has been measured, it should be noted here that the time of this measurement is not particularly limited. For example, the urine salt contained within the second urine expelled in the early morning or within urine expelled at night may be measured.

Furthermore, although the tentative points are calculated using the measurement results and measurement frequency, the present invention is not limited to such a setup. For example, the tentative points may be calculated using only the measurement frequency.

In addition, in the case where the degree of divergence is determined to be high, the acquired tentative points are applied directly as finalized points, and in the case where the degree of divergence is determined to be low, two times the amount of acquired tentative points are applied as finalized points; however, the present invention is not limited to such a setup. In other words, another method may be used as long as it awards higher amounts of points the lower the degree of divergence is.

In addition, here, the tentative points are revised based on the degree of divergence between a value estimated from the first biological information (urine salt content) and the second biological information (blood pressure); however, the present invention is not limited to such a setup. That is, a target value storage means, which stores a target value for the second biological information, may be included in the server apparatus 3, and the tentative points may be revised based on the degree to which this target value has been approached. Through this, it is possible to provide the patient with higher incentives when daily lifestyle improvement has a positive effect on treatment. Therefore, it can be expected that the patient's motivation in regards to improving his/her lifestyle will increase. In other words, by employing a system which awards higher amounts of finalized points for higher degrees of approaching the target value for the second biological information, it can be expected that the patient will become more enthusiastic about improving his/her lifestyle.

In addition, a means for creating the advice and tentative points based on the results of the measurement taken via the measurement unit 12 may be present within the client terminal 1. With such a setup, the client terminal 1 does not need to send information to the server apparatus 3 every time a measurement is taken. In other words, communication between the client terminal 1 and the server apparatus 3 can be performed only when necessary (for example, only when an extremely abnormal reading is taken or during a periodic results check by the server apparatus 3), which lightens the burden on the server apparatus 3. Moreover, as the client terminal 1 requires no time for communications with the server apparatus 3, advice can be obtained with less delay.

In addition, the configuration described here is one in which the frequency of salt content measurements is calculated within the client terminal 1, written in the information storage unit 16, then sent from the sending/receiving unit 15 to the server apparatus 3 and stored in the biological information storage unit 350; however, the present invention is not limited to such a configuration. For example, the configuration may be one in which the information sent from the client terminal 1 to the server apparatus 3 is only the salt content measurement results and the measurement time, and the number of measurements within a set period is determined by the incentive information creation unit 320 of the server apparatus 3.

In addition, a clinic is given as an example of the third-party institution, but a clinic is only one example. The third-party institution referred to here includes health centers, health management centers, laboratory test centers, as well as, of course, clinics and hospitals. Therefore, the information reported by the third-party institution is objective and reliable, and thus the incentive information can be awarded with accuracy.

Furthermore, the users of the present system are called "patients," but the "patients" referred to here includes persons who do not actually require treatment. In other words, as long as a person makes use of the third-party institution, he/she is equivalent to the "patient" described here.

### (Second Embodiment)

In the above first embodiment, information is sent/received between a client terminal and a clinic terminal via a network; however, in the present second embodiment, information is sent/received between the client terminal and the clinic terminal via a storage medium. In other respects, the configuration is the same as that of the first embodiment.

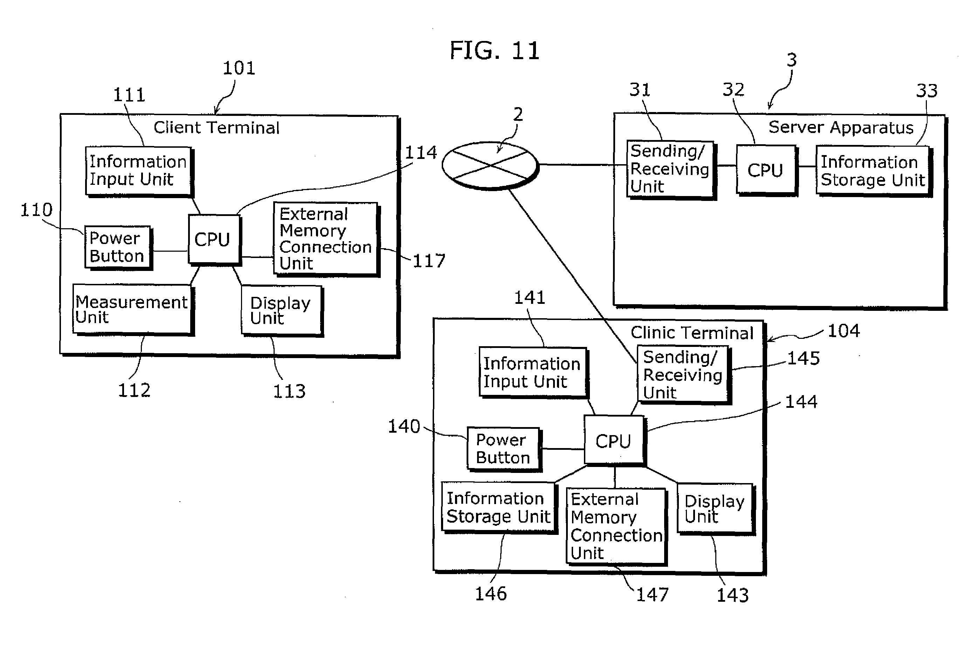

FIG. 11 is a block diagram showing a simplified configuration of the lifestyle improvement support system according to the second embodiment of the present invention. A client terminal 101 has, in place of the information storage unit 16, an external memory connection unit 117, which can read/write information to a non-volatile external memory, such as an SD card. The external memory connection unit 117 corresponds to the first biological information sending unit of the present invention. A clinic terminal 104 also has an external memory connection unit 147, which can read/write information to a non-volatile external memory, such as an SD card. The client terminal 101 is not connected to a communications network 2, such as the Internet. The server apparatus 3 and the clinic terminal 104 are connected via the communications network 2, such as the Internet. In other respects, the configuration is the same as that of the first embodiment.

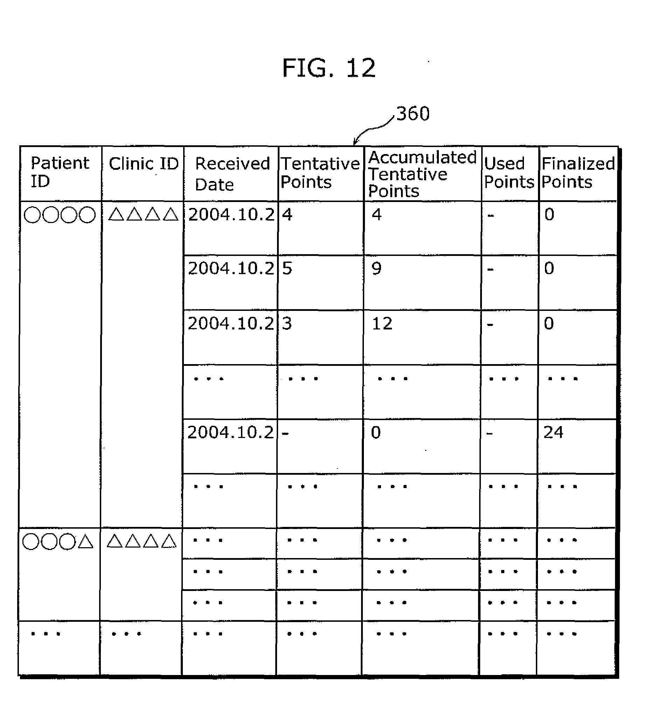

FIG. 12 is a diagram showing an example of data stored in a points/bills storage unit 360 in the second embodiment of the present invention. Aside from the received dates all being identical (October 2, 2004), this diagram is the same as FIG. 5, described in the first embodiment. The reason the received dates are identical is because the processings are performed all at once on the day when the SD card has been brought to the clinic; details shall be given later.

It should be noted that the present embodiment describes a situation in which the server apparatus 3 and the clinic terminal 10 are connected via the communications network 2 such as the Internet; however, the server apparatus 3 may be installed within the clinic. In such a case, the server apparatus 3 and the clinic terminal 10 are connected via a LAN set up within the clinic.

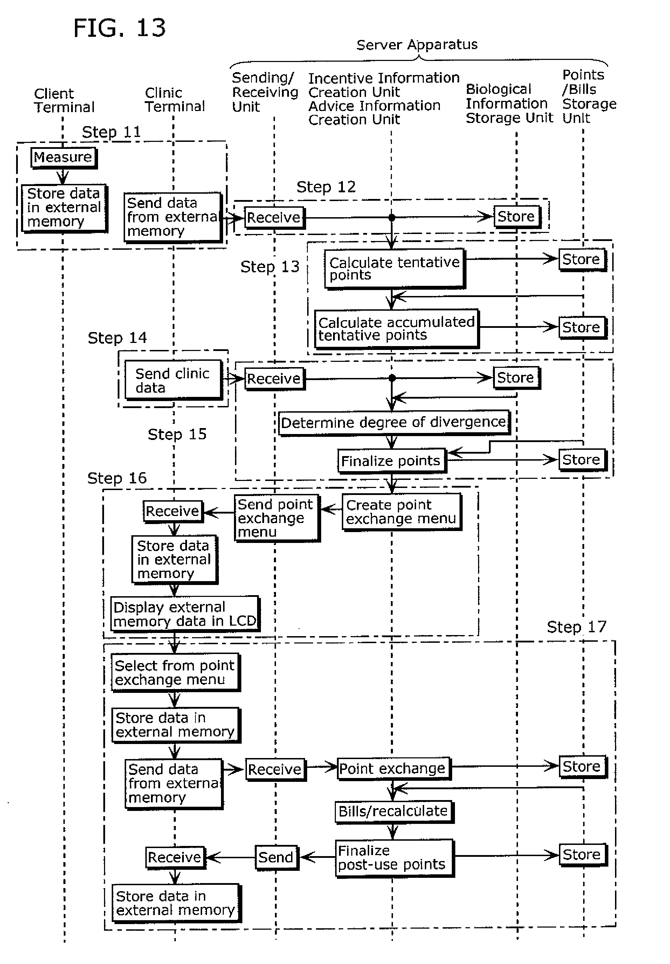

FIG. 13 is a flowchart showing a process of the lifestyle improvement support system according to the second embodiment of the present invention. In the following descriptions, detailed descriptions of processes identical to those of the first embodiment shall be omitted.

First, an external memory, such as an SD card, is connected to the external memory connection unit 117 of the client terminal. When the patient uses the client terminal 101 and measures his/her urine salt content, the measurement results are stored in the SD card via the external memory connection unit 117. The patient removes the SD card from the client terminal and brings it with him/her when going to the clinic. When the SD card is connected to the external memory connection unit 147 of the clinic terminal 104, the measurement results stored within the SD card are sent from the clinic terminal 104 to the server apparatus 3 (Step 11). The server apparatus 3 calculates tentative points and the accumulated tentative points based on the measurement results received from the clinic terminal 104 (Steps 12 and 13).

In addition, when the patient's blood pressure reading is inputted into the clinic terminal 104, that blood pressure reading is sent from the clinic terminal 104 to the server apparatus 3 (Step 14). The server apparatus 3 finds the degree of divergence between the blood pressure reading received from the clinic terminal 104 and the estimated reading, and calculates the finalized points by revising the tentative points based on the degree of divergence found (Step 15). Then, a menu of incentives that can be exchanged for the finalized points is created.

Here, the point exchange menu in the present second embodiment is sent from the server apparatus 3 to the clinic terminal 104, is received by the sending/receiving unit 115 of the clinic terminal 104, and is stored in the SD card by the external memory connection unit 147 of the clinic terminal 104. After this, when the point exchange menu is read out from the SD card by the external memory connection unit 147 of the clinic terminal 104, a notification screen showing the finalized points and incentives is displayed in the display unit 143 of the clinic terminal 104 (Step 16). This notification screen is the same as that shown in FIG. 7 of the first embodiment, and thus descriptions shall hereby be omitted.

The patient uses an information input unit 141 to select the incentive he/she desires from the point exchange menu displayed in the display unit 143 of the clinic terminal 104. Information indicating the desired incentive is stored in the SD card by the external memory connection unit 147 of the clinic terminal 104, as well as being sent to the server apparatus 3. As a result, the patient can thus enjoy an incentive that corresponds to the finalized points he/she acquired. After this, the finalized points remaining after the incentive has been received are sent from the server apparatus 3 to the clinic terminal 104, and are stored in the DS card by the external memory connection unit 147 of the clinic terminal 104 (Step 17). Through this, a notification screen showing the incentive is displayed in the display unit 143 of the clinic terminal 104. This notification screen is the same as that shown in FIG. 9 of the first embodiment, and thus descriptions shall hereby be omitted.

As described thus far, the lifestyle improvement support system of the present second embodiment uses not only biological information sent from the client terminal 1 but also objective biological information sent from the clinic terminal 4 to create incentive information, thus making it possible to accurately award the incentive information, in the same manner as in the first embodiment. In addition, information is sent/received between the client terminal 1 and the clinic terminal 4 via an SD card, which makes it possible for the patient to use the present system even in the case where the client terminal 1 is not connected to a network.

It should be noted here that a setup in which the tentative points and advice are not outputted (are not presented to the patient) has been described, but the present invention is not limited to such a setup. In other words, the tentative points and advice information may be outputted, in the same manner as in the first embodiment, even in the case where information is sent/received between the client terminal and the clinic terminal via a storage medium.

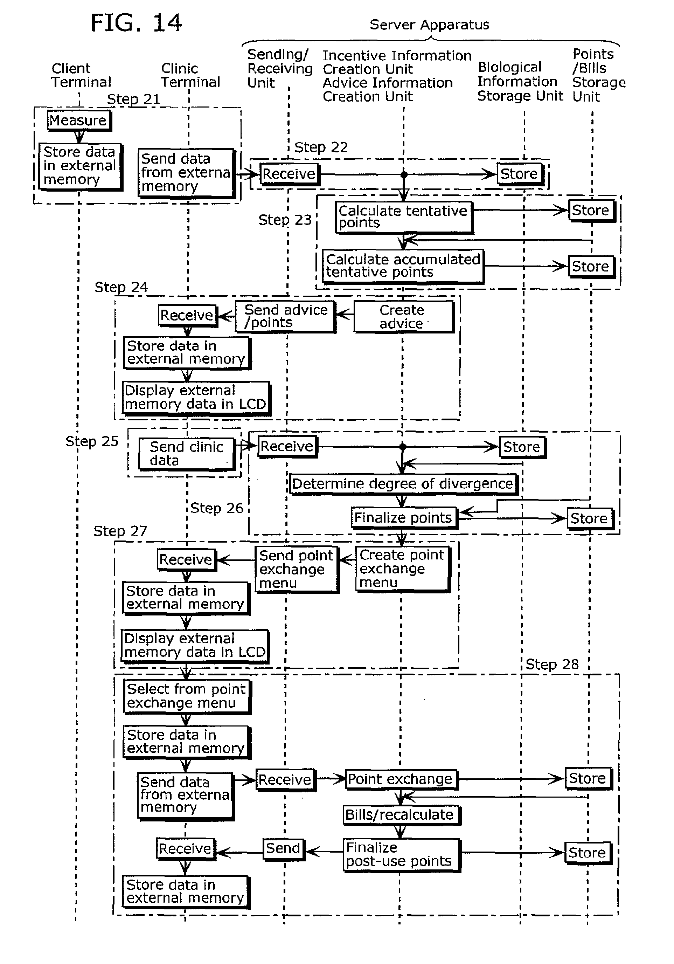

FIG. 14 is a flowchart showing a process of another lifestyle improvement support system according to the second embodiment of the present invention. The only difference from FIG. 13 is that Step 24, which includes the creation of advice information up to the display of a notification screen, has been added. In other words, the server apparatus 3 creates the advice information in the same manner as in the first embodiment, and sends that advice information and the tentative points to the clinic terminal 104. Through this, the external memory connection unit 147 of the clinic terminal 104 records the advice information and tentative points in the SD card, and a notification screen showing the tentative points and advice is displayed in the display unit 143 of the clinic terminal 104. This notification screen is the same as that shown in FIG. 6 of the first embodiment, and thus descriptions shall hereby be omitted.

As has been described thus far, the tentative points and advice information can be displayed in the display unit 143 of the clinic terminal 104, in the same manner as in the first embodiment, even in the case where information is sent/received between the client terminal and the clinic terminal via a storage medium. Through this, the patient can instantly know his/her points and advice, in the same manner as the first embodiment. However, while the setup that displays various screens in the display unit 143 of the clinic terminal 104 is advantageous in that the patient can instantly know his/her points and advice, this setup is disadvantageous in that the burden of processing performed within the clinic increases. Accordingly, the various screens may be displayed in a display unit 113 of the client terminal 101 when the patient brings the SD card home and connects it to the external memory connection unit 117 of the client terminal 101.

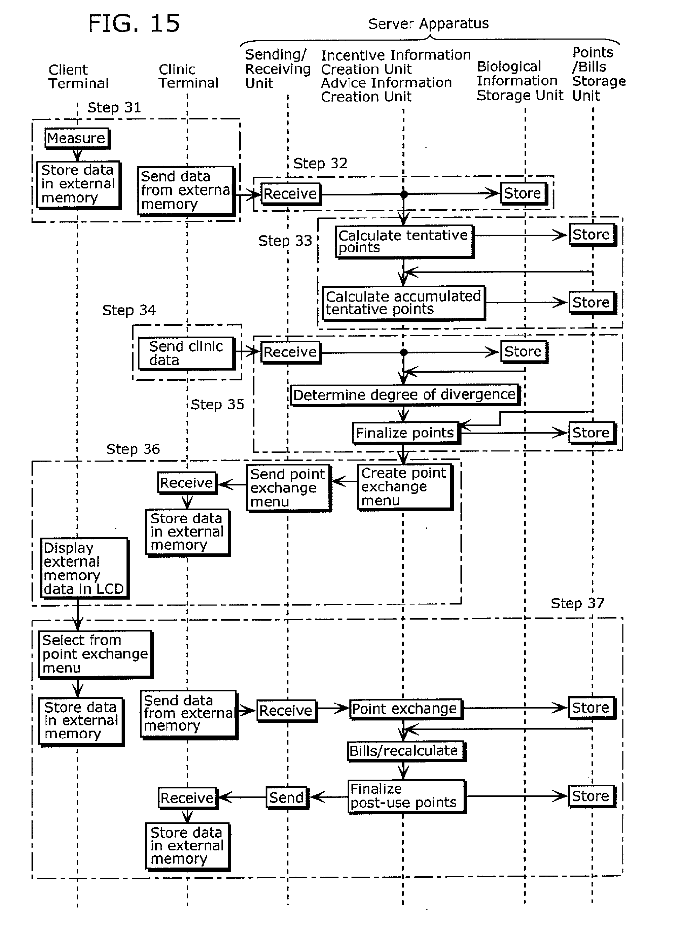

FIG. 15 is a flowchart showing a process of another lifestyle improvement support system according to the second embodiment of the present invention. The difference from FIG. 13 is that the various screens are displayed not in the display unit 143 of the clinic terminal 104 but in the display unit 113 of the client terminal 101.

In other words, as in FIG. 13, the point exchange menu created by the server apparatus 3 is stored in the SD card by the external memory connection unit 147 of the clinic terminal 104. Here, the clinic passes the SD card to the patient. Through this, when the patient brings the SD card home and connects it to the external memory connection unit 117 of the client terminal 101, a notification screen showing the finalized points and incentives is displayed in the display unit 113 of the client terminal 101 (Step 36). This notification screen is the same as that shown in FIG. 7 of the first embodiment, and thus descriptions shall hereby be omitted.

Then, the patient uses an information input unit 111 to select the incentive he/she desires from the point exchange menu displayed in the display unit 113 of the client terminal 104. Information indicating the desired incentive is stored in the SD card by the external memory connection unit 117 of the client terminal 101. In addition, in the case where the patient takes his/her daily measurements using the measurement unit 112, the measurement results are also stored in the SD card via the external memory connection unit 117 of the client terminal 101.

The patient removes the SD card from the client terminal and brings it with him/her when going to the clinic, as he/she did the previous time. Then, when the SD card is connected to the external memory connection unit 147 of the clinic terminal 104, the information stored within the SD card is sent from the clinic terminal 104 to the server apparatus 3 (Step 31). Here, information indicating the desired incentive and the measurement results from the interval between the previous examination and the present examination are included in the information sent to the server apparatus 3. Through this, the server apparatus 3 calculates tentative points and the accumulated tentative points based on the measurement results received from the clinic terminal 104 (Steps 32 and 33).

In addition, when the patient's blood pressure reading is inputted into the clinic terminal 104, that blood pressure reading is sent from the clinic terminal 104 to the server apparatus 3 (Step 34). The server apparatus 3 finds the degree of divergence between the blood pressure reading received from the clinic terminal 104 and the estimated reading, and calculates the finalized points by revising the tentative points based on the degree of divergence found (Step 35). Then, a menu of incentives that can be exchanged for the finalized points is created. This point exchange menu is sent from the server apparatus 3 to the clinic terminal 104, is received by the sending/receiving unit 115 of the clinic terminal 104, and is stored in the SD card by the external memory connection unit 147 of the clinic terminal 104. Here, the clinic passes the SD card to the patient. Through this, when the patient brings the SD card home and connects it to the external memory connection unit 117 of the client terminal 101, a notification screen showing the finalized points and incentives is displayed in the display unit 113 of the client terminal 101 (Step 36).

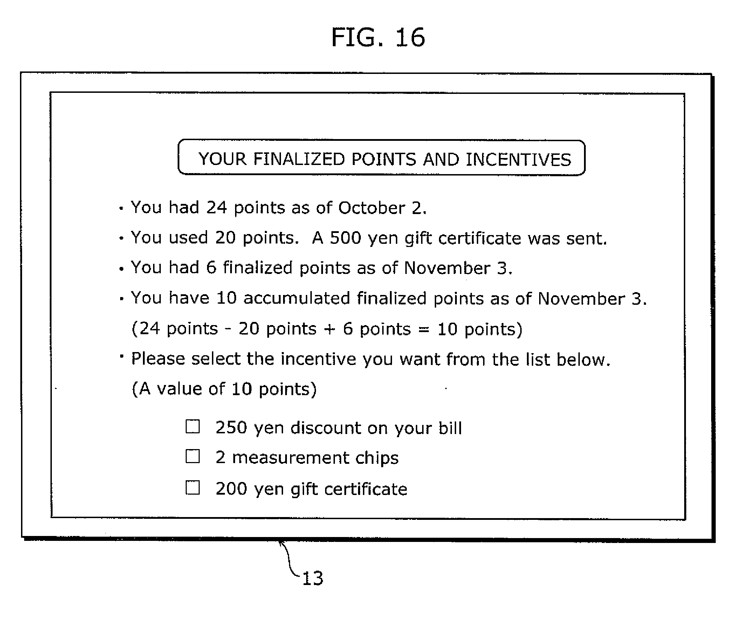

FIG. 16 is a diagram showing an example of a notification screen that shows finalized points and incentives. Incentives and fluctuation in finalized points are included in this notification screen. The reason the finalized points have fluctuated is because the processings are performed all at once on the day when the SD card has been brought to the clinic. In other words, finalized points decrease when an incentive is chosen through the client terminal 101, but that processing is performed by the server apparatus 3 upon the patient's next visit to the clinic, when he/she brings the SD card. Upon the patient's next visit to the clinic, when he/she brings the SD card, his/her blood pressure is measured, in the same manner as the previous time, and thus new finalized points are issued. Therefore, as mentioned earlier, the finalized points fluctuate when the patient brings the SD card home from the clinic and connects it to the external memory connection unit 117 of the client terminal 101.

As described thus far, it is possible to display various screens in the display unit 113 of the client terminal 101 rather than the display unit 143 of the clinic terminal 104 even in the case where information is sent/received between the client terminal and the clinic terminal via a storage medium. Accordingly, it is no longer necessary to display the various screens in the display unit 143 of the clinic terminal 104, and therefore the disadvantage in which the burden of processing within the clinic does not arise.

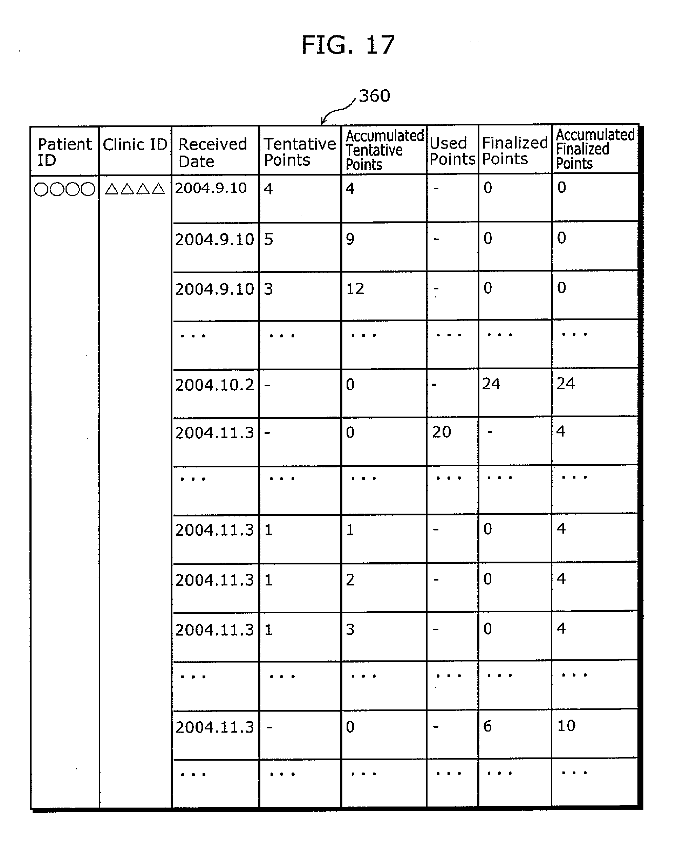

Naturally, with this setup, the patient cannot know his/her points in real time; however, as the fluctuation in finalized points is notified to the patient at the same time as the incentives, confusion experienced by the patient can be avoided. The accumulated finalized points may be included in this notification screen. In this case, the accumulated finalized points can be obtained by totaling the finalized points stored in the points/bills storage unit 360. Alternatively, a configuration in which the accumulated finalized points are also stored in the points/bills storage unit 360 in addition to the finalized points may be employed, as shown in FIG. 17.

Although a notification screen showing the finalized points and incentives is displayed in Step 36, it should be noted that the present invention is not limited to such a setup. That is, a notification screen showing the finalized points and advice may be displayed in Step 36.

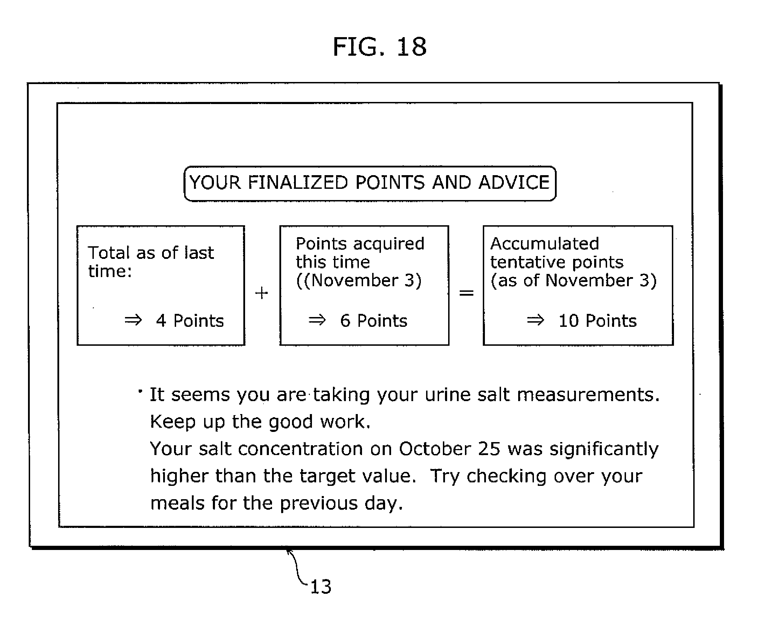

FIG. 18 is a diagram showing an example of the notification screen that shows the finalized points and advice. The accumulated finalized points acquired as of the last time, the finalized points acquired this time, the total of these, which is the current amount of accumulated finalized points, and the advice information are included in this notification screen. In other words, along with the finalized points, it is possible to display advice information created based on the tentative points in the display unit 113 of the client terminal 101. The accumulated tentative points acquired thus far may also be included in this notification screen. As mentioned earlier, with such a setup, evaluation is performed without negating the patient's effort to improve his/her lifestyle, which has the effect of causing the patient to continue his/her lifestyle improvement. It goes without saying that such a configuration, which displays the advice information along with the finalized points, can be utilized in the first embodiment as well.

In addition, although an SD card has been given as an example of the storage medium, it should be noted that the storage medium is not limited to SD cards. In other words, any storage medium, such as a miniSD card, a Memory Stick, a Compact Flash device, a Microdrive device, an xD-Picture Card, or a USB-based memory, may be used as long as it is a portable non-volatile memory.

Furthermore, while the configuration described here is one in which an external memory connection unit is present in the clinic terminal 104, the present invention is not limited to such a configuration. For example, in the case where the server apparatus 3 and the clinic terminal 104 are installed within the clinic, the configuration may be one in which the external memory connection unit is present in the server apparatus 3. In such a case, when the SD card is connected to the external connection memory unit of the server apparatus 3, it is possible to read out the information recorded in the SD card by the client terminal 101 directly into the server apparatus 3.

### (Third Embodiment)

The configuration described in the first embodiment awards points to patients; however, the present third embodiment employs a configuration in which points are awarded to the third-party institution such as a clinic in addition to the patient. In other words, the incentive information creation unit 320 of the present third embodiment creates incentive information for the third-party institution as well as for the patient. The third-party institution incentive information is information for increasing the incentives for efforts on the part of the third-party institution. In other respects, the configuration is the same as that of the first embodiment.

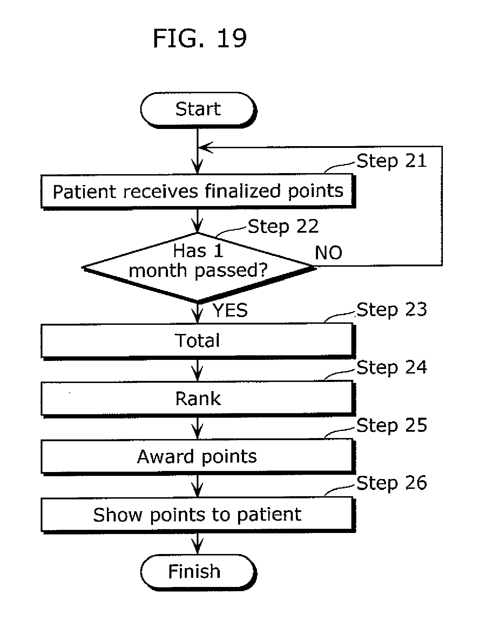

FIG. 19 is a flowchart showing a process in which a lifestyle improvement support system according to the third embodiment of the present invention awards points to a clinic.

First, each step described in the first embodiment is performed. That is, when a patient who is examined at a clinic engages in lifestyle improvement activities at home, information thereof is sent to the lifestyle improvement support system. Through this, the patient obtains finalized points calculated based on the results of examination at the clinic. As a result, finalized points are stored on a patient-by-patient basis, as shown in FIG. 5 (Step 21).

Accordingly, the incentive information creation unit 320 of the server apparatus 3 totals the number of finalized points for each clinic ID every set intervals of time (for example, one month) (Step 22 and 23). It can be said that clinics with large numbers of finalized points are clinics either with a large number of patients who are making effort to improve their lifestyle or with a large number of patients who are actually improving their lifestyle.

Therefore, the incentive information creation unit 320 of the server apparatus 3 ranks the clinics based on the total number of finalized points each clinic has acquired. For example, when clinic points are awarded in the amount of five points for the clinic in first place, four points for the clinic in second place, three points for the clinic in third place, two points for the clinic in fourth place, and one point for the clinic in fifth place (Step 25), those figures are stored in the points/bills storage unit 360 in a different format.

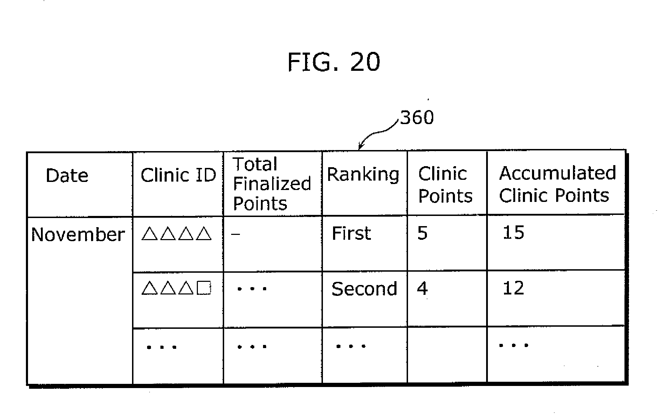

FIG. 20 is a diagram showing an example of data stored in the points/bills storage unit 360. As shown in FIG. 20, the points/bills storage unit 360 stores the date, clinic ID, total number of finalized points, rank, and clinic points in association with one another. The rank shown here is that for the month of November. In this diagram, the clinic with an ID of "triangle, triangle, triangle, triangle", which has acquired fifteen accumulated clinic points, is in first place, whereas the clinic with an ID of "triangle, triangle, triangle, square", which has acquired twelve accumulated clinic points, is in second place.

Once the ranking is complete, the results are shown to the patient (Step 26). There are various methods of showing the results to the patient, such as over the Internet, and there is no particular limitation.

As has been described thus far, the present third embodiment utilizes a configuration in which points are awarded to clinics as well as to patients, and the clinic points can be shown to the patients. Through this, it is possible to objectively promote the efforts of clinics in regards to lifestyle improvement, which has the effect of reducing the tendency of patients to avoid clinics by cancelling examinations and so on.

Note that a discount or the like on the clinic's fees for utilizing the present system may be introduced in the case where the clinic points exceed a certain amount. It can be expected that doing so will make it easier for medical institutions to work towards lifestyle improvement.

In the case of implementing a lifestyle improvement service using this system, the following kinds of service business planned out by business units can be considered in the implementation. That is, assume that the service provider who operates the server apparatus 3 is an insurance company; the service provided to the patient is life insurance, and the incentive is cash back on the insurance premium. In such a case, it is preferable for the service provider, or the insurance company, to issue points to the third-party institution, or the medical institution, as an incentive based on the degree of effectiveness. With such a setup, it is possible to present the degree of quality of the third-party institution to the patient by publicizing the points, which has the effect of making the medical institution more public and recruiting more patients.

### Industrial Applicability

The lifestyle improvement support system according to the present invention is applicable as a system for supporting improvement in the lifestyle of patients of lifestyle-related diseases (particularly sufferers of hypertension) which requires incentive information to be awarded in an accurate manner.

## Claims

1. A lifestyle improvement support system which supports improvements in lifestyle, said system comprising:
a first biological information acquisition unit operable to acquire first biological information, which is biological information of a patient and which is reported by the patient;
a second biological information acquisition unit operable to acquire second biological information, which is biological information of the patient and is reported by a third-party institution, and which has a correlation with the first biological information;
an incentive information creation unit operable to create incentive information, which is information for increasing the incentive for the patient to improve his/her lifestyle, based on the first biological information and the second biological information; and
an output unit operable to output the incentive information.

2. The lifestyle improvement support system according to Claim 1,
wherein said incentive information creation unit is operable to create tentative incentive information, which is the incentive information that has not yet been finalized, based on the first biological information, and after that, create finalized incentive information, which is the incentive information that has been finalized, based on the tentative incentive information and the second biological information.

3. The lifestyle improvement support system according to Claim 2, further comprising
a correlative relationship storage unit operable to store a correlative relationship between the first biological information and the second biological information,
wherein said incentive information creation unit is operable to find an estimated value of the second biological information based on the correlative relationship and the first biological information, and create the finalized incentive information so that the incentive for the patient to improve his/her lifestyle increases the lower the degree of divergence between the second biological information and the estimated value of the second biological information.

4. The lifestyle improvement support system according to Claim 3, further comprising
a target value storage unit operable to store a target value of the second biological information,
wherein said incentive information creation unit is operable to create the finalized incentive information so that the incentive for the patient to improve his/her lifestyle increases the higher the degree of achievement between the second biological information and the target value of the second biological information.

5. The lifestyle improvement support system according to Claim 3, further comprising
a biological information storage unit operable to store the second biological information,
wherein said incentive information creation unit is operable to create the finalized incentive information so that the incentive for the patient to improve his/her lifestyle increases the higher the degree of improvement between the present second biological information and the previous second biological information.

6. The lifestyle improvement support system according to Claim 1, further comprising
an advice information creation unit operable to create advice information, which is information indicating advice for the patient regarding improvement in his/her lifestyle, based on the first biological information,
wherein said output unit is operable to output the advice information.

7. The lifestyle improvement support system according to Claim 1,
wherein said incentive information creation unit is operable to generate third-party institution incentive information, which is information for increasing the incentive for efforts on the part of the third-party institution, based on the incentive information, and
said output unit is operable to output the third-party institution incentive information.

8. The lifestyle improvement support system according to Claim 1, further comprising
a client terminal which belongs to the patient; and
a third-party institution terminal which belongs to the third-party institution,
wherein said client terminal includes:
a first biological information acceptance unit operable to accept input of the first biological information; and
a first biological information sending unit operable to send the first biological information accepted by said first biological information acceptance unit to said first biological information acquisition unit via a network, and
said third-party terminal includes:
a second biological information acceptance unit operable to accept input of the second biological information; and
a second biological information sending unit operable to send the second biological information accepted by said second biological information acceptance unit to said second biological information acquisition unit via a network.

9. The lifestyle improvement support system according to Claim 1, further comprising
a client terminal which belongs to the patient; and
a third-party institution terminal which belongs to the third-party institution,
wherein said client terminal includes:
a first biological information acceptance unit operable to accept input of the first biological information; and
a first biological information sending unit operable to send the first biological information accepted by said first biological information acceptance unit to said first biological information acquisition unit via a storage medium, and
said third-party terminal includes:
a second biological information acceptance unit operable to accept input of the second biological information; and
a second biological information sending unit operable to send the second biological information accepted by said second biological information acceptance unit to said second biological information acquisition unit via a network.

10. The lifestyle improvement support system according to Claim 1,
wherein the first biological information is information that indicates results of the patient's lifestyle improvement.

11. A lifestyle improvement support method for supporting improvements in lifestyle, said method comprising:
a first biological information acquisition step of acquiring first biological information, which is biological information of a patient and which is reported by the patient;
a second biological information acquisition step of acquiring second biological information, which is biological information of the patient and is reported by a third-party institution, and which has a correlation with the first biological information;
an incentive information creation step of creating incentive information, which is information for increasing the incentive for the patient to improve his/her lifestyle, based on the first biological information and the second biological information; and
an output step of outputting the incentive information.

12. The lifestyle improvement support method according to Claim 11,
wherein said incentive information creation step includes creating tentative incentive information, which is the incentive information that has not yet been finalized, based on the first biological information, and after that, creating finalized incentive information, which is the incentive information that has been finalized, based on the tentative incentive information and the second biological information.

13. The lifestyle improvement support method according to Claim 12,
wherein said incentive information creation step includes: finding an estimated value of the second biological information, based on a correlative relationship between the first biological information and the second biological information, and the first biological information; and creating the finalized incentive information so that the incentive for the patient to improve his/her lifestyle increases the lower the degree of divergence between the second biological information and the estimated value of the second biological information.

14. The lifestyle improvement support method according to Claim 11, further comprising
an advice information creation step of creating advice information, which is information indicating advice for the patient regarding improvement in his/her lifestyle, based on the first biological information,
wherein said output step includes outputting the advice information.

15. The lifestyle improvement support method according to Claim 11,
wherein said incentive information creation step includes generating third-party institution incentive information, which is information for increasing the incentive for efforts on the part of the third-party institution, based on the incentive information for increasing the incentive for the patient to improve his/her lifestyle, and
said output step includes outputting the third-party institution incentive information.

16. A program for supporting improvements in lifestyle, said program causing a computer to execute:
a first biological information acquisition step of acquiring first biological information, which is biological information of a patient and which is reported by the patient;
a second biological information acquisition step of acquiring second biological information, which is biological information of the patient and is reported by a third-party institution, and which has a correlation with the first biological information;
an incentive information creation step of creating incentive information, which is information for increasing the incentive for the patient to improve his/her lifestyle, based on the first biological information and the second biological information; and
an output step of outputting the incentive information.
